# EUROPÄISCHE PATENTSCHRIFT

(11) **EP 1 761 536 B1**
(45) Veröffentlichungstag und Bekanntmachung des Hinweises auf die Patenterteilung: **26.12.2007**
(21) Anmeldenummer: 05751714.6
(22) Anmeldetag: 15.06.2005
(51) Int. Cl.: C07D 475/00, A61K 31/519, A61P 35/00

(54) **NEUE 2-BENZYLAMINODIHYDROPTERIDINONE, VERFAHREN ZU DEREN HERSTELLUNG UND DEREN VERWENDUNG ALS ARZNEIMITTEL**
NOVEL 2-BENZYLAMINODIHYDROPTERIDINONES, METHOD FOR PRODUCING THEM AND USE THEREOF AS DRUGS
NOUVEAUX 2-BENZYLAMINODIHYDROPTERIDINONES, PROCEDE DE PRODUCTION ASSOCIE ET LEUR UTILISATION EN TANT QUE MEDICAMENTS

(30) Priorität: 21.06.2004 DE 102004029784
(43) Veröffentlichungstag der Anmeldung: 14.03.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: HOFFMANN, Matthias, 88441 MITTELBIBERACH (DE); STEEGMAIER, Martin, 72762 Reutlingen (DE); GRAUERT, Matthias, 88400 BIBERACH (DE); LEHMANN-LINTZ, Thorsten, 88416 OCHSENHAUSEN (DE); REDEMANN, Norbert, 88400 BIBERACH (DE); SOLCA, Flavio, A-1230 WIEN (AT)
(74) Vertreter: Hammann, Heinz
(86) Internationale Anmeldenummer: PCT/EP2005/006404
(87) Internationale Veröffentlichungsnummer: WO 2005/123736

(56) Entgegenhaltungen:
- WO-A-01/19825
- WO-A-03/020722
- DONALD E. WOLF, R. CHRISTIAN ANDERSON, EDWARD A. KACZKA, STANTON A. HARRIS, GLEN E. ARTH ET AL.: "The Structure of Rhizopterin" J. AM. CHEM. SOC., Bd. 69, 1947, Seiten 2753-2759, XP002352205

## Beschreibung

Die vorliegende Erfindung betrifft neue 2-Benzylaminodihydropteridinone der allgemeinen Formel (I) 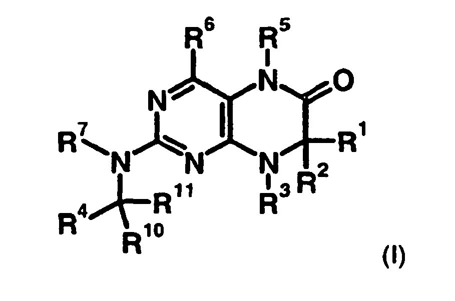 wobei die Reste R¹ bis R⁷, R¹⁰ und R¹¹ die in den Ansprüchen und der Beschreibung genannten Bedeutungen haben, deren Isomere, Verfahren zur Herstellung dieser Dihydropteridinone sowie deren Verwendung als Arzneimittel.

### Hintergrund der Erfindung

Pteridinon-Derivate sind als Wirkstoffe mit antiproliferativer Wirkung aus dem Stand der Technik bekannt. WO 01/019825 und WO 03/020722 beschreiben die Verwendung von Pteridinonderivaten zur Behandlung von Tumorerkrankungen.

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, Cell 102:279-91). Ein spezifischer Zellzyklusarrest in der G2/M Phase kann aber auch z.B. durch Inhibition von spezifischen Phosphatasen wie z.B. Cdc25C (Russell and Nurse 1986, Cell 45:145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, Cell 49:559-67). Desweiteren kann ein Arrest in der G2/M Phase auch durch Inhibition von bestimmten Motorproteinen, den so genannten Kinesinen wie z.B. Eg5 (Mayer et al. 1999, Science 286:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, Proc NatI Acad Sci U S A 77:1561-5).

Neben den Cyclin-abhängigen und den Aurora Kinasen spielen des weiteren die so genannten Polo-like Kinasen, eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. 1998, Genes Dev. 12:3777-87; Qian et al. 2001, Mol Biol Cell. 12:1791-9). Die Injektion von PLK-1 Antikörpern führt zu einem G2 Arrest in nicht transformierten Zellen, während Tumorzellen in der Mitosephase arretieren (Lane und Nigg 1996, J Cell Biol. 135:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, Oncogene 14 :543 -549; Knecht etal. 1999, Cancer Res. 59:2794 -2797; Wolf et al. 2000, Pathol. Res. Pract. 196:753 -759; Takahashi et al. 2003, Cancer Sci. 94:148-52) gezeigt werden. Daher stellt diese Klasse von Proteinen ebenfalls einen interessanten Angriffspunkt zur therapeutischen Intervention proliferativer Krankheiten dar (Liu and Erikson 2003, Proc Natl Acad Sci U S A 100:5789-5794).

Die Resistenz vieler Tumorarten erfordert die Entwicklung neuer Arzneimittel zur Tumorbekämpfung.

Es ist die Aufgabe der vorliegenden Erfindung neue Verbindungen mit antiproliferativer Wirkung bereitzustellen.

### Detaillierte Beschreibung der Erfindung

Überraschenderweise wurde gefunden, dass Verbindungen der allgemeinen Formel (I), worin die Reste R¹ bis R⁷, R¹⁰ und R¹¹ die nachstehend genannten Bedeutungen haben, als Inhibitoren spezifischer Zellzykluskinasen, insbesondere der Polo-like Kinasen, wirken. Die genannten Verbindungen zeigen antiproliferative Wirkung, indem sie Zellen in der Mitosephase des Zellzykluses arretieren bevor der programmierte Zelltod in den arretierten Zellen eingeleitet wird. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft daher Verbindungen der allgemeinen Formel (I) 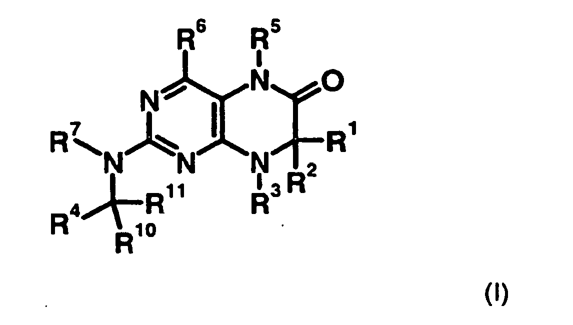 worin
R¹, R² gleich oder verschieden, ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl,
C₃-C₈-Heterocycloalkyl, -X-Aryl, -X-Heteroaryl, -X-Cycloalkyl, -X-Heterocycloalkyl, -NR⁸-Aryl, -NR⁸-Heteroaryl, -NR⁸-Cycloalkyl, und -NR⁸-Heterocycloalkyl,
   oder
ein Rest ausgewählt aus der Gruppe bestehend aus Wasserstoff, Halogen, COXR⁸, CON(R⁸)₂, COR⁸ und XR⁸,
   oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl, Aryl, Heteroaryl , C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl oder R¹ und R³ oder R² und R³ gemeinsam eine gesättigte oder ungesättigte C₃-C₄-Alkylbrücke, die 1 bis 2 Heteroatome enthalten kann,
R⁴ gegebenenfalls substituiertes Aryl oder Heteroaryl,
R⁵ ein Rest ausgewählt aus der Gruppe bestehend aus Wasserstoff, CHO, XH, -X-C₁-C₂-Alkyl und einer gegebenenfalls substituierten C₁-C₃-AlkylGruppe,
R⁶ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
R⁷ Wasserstoff oder -CO-C₁-C₄-Alkyl, -CO-NH-C₁-C₄-Alkyl, -CO-X-C₁-C₄-Alkyl, und
X jeweils unabhängig voneinander, O oder S,
R⁸ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl, Benzyl und Phenyl,
R¹⁰ und R¹¹ jeweils unabhängig voneinander, Wasserstoff oder ein Rest ausgewählt aus der Gruppe aus gegebenenfalls substituiertem C₁-C₄-Alkyl,
oder R¹⁰ und R¹¹ gemeinsam eine 2-5 gliedrige Alkylbrücke,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate, bedeuten.

Bevorzugt sind Verbindungen der Formel (I),worin
R¹ bis R⁴ die angegebene Bedeutung aufweisen,
R⁵ Methyl, und
R⁶ und R⁷ Wasserstoff,
R¹⁰ und R¹¹ jeweils unabhängig voneinander, Wasserstoff oder Methyl,
   oder
R¹⁰ und R¹¹ gemeinsam Cyclopropyl,
bedeuten.

Weiterhin bevorzugt sind Verbindungen der Formel (I), worin
R³ bis R⁷, R¹⁰ und R"die oben genannten Bedeutungen aufweisen und
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
   oder
R¹ und R² gemeinsam eine 2- bis 5-gliedrige Alkylbrücke,
bedeuten.

Besonders bevorzugt sind Verbindungen der Formel (I), worin
R¹, R³, R⁴ bis R⁷, R¹⁰ und R¹¹ die oben genannten Bedeutungen aufweisen, und
R³ gleich Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl enthält.

Weiterhin besonders bevorzugt sind Verbindungen der Formel (I), worin
R¹ bis R³, R⁵ bis R⁷, R¹⁰ und R¹¹ die oben genannten Bedeutungen aufweisen, und
R⁴ ein Rest der allgemeinen Formel 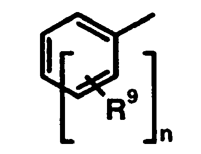
R⁹ gleich oder verschieden, ein Rest ausgewählt aus der Gruppe bestehend aus gegebenenfalls substituiertem C₁-C₆-Alkyl, C₂-C₆-Alkenyl, C₂-C₆-Alkinyl, -O-C₁-C₆-Alkyl, -O-C₂-C₆-Alkenyl, -O-C₂-C₆-Alkinyl, C₃-C₆-Heterocycloalkyl, C₃-C₆-Cycloalkyl, Aryl, Heteroaryl, -O-Aryl, -O-Heteroaryl, -O-Cycloalkyl, und -O-Heterocycloalkyl oder
ein Rest ausgewählt aus der Gruppe bestehend aus Wasserstoff, -CONH₂, - COOR⁸, -OCON(R⁸)₂, -N(R⁸)₂, -NHCOR⁸,-NHCON(R⁸)₂ , -NO₂, CF₃ , Halogen, - O-C₁-C₆-Alkyl-Q¹, -CONR⁸-C₁-C₁₀-Alkyl-Q¹, -CONR⁸-C₂-C₁₀-Alkenyl-Q¹, -CONR⁸-Q², Halogen, OH, -SO₂R⁸, -SO₂N(R⁸)₂, -COR⁸, -COOR⁸, -N(R⁸)₂, - NHCOR⁸, -CONR⁸OC₁-C₁₀ -Alkyl-Q¹ und CONR⁸O-Q²,
R⁸ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl und Phenyl,
Q¹ Wasserstoff, -NHCOR⁸, oder ein Rest ausgewählt aus der Gruppe bestehend aus einer gegebenenfalls substituierten -NH-Aryl-, -NH-Heteroaryl-, Aryl-, Heteroaryl-, C₃-C₈-Cycloalkyl- und Heterocycloalkyl-Gruppe,
Q² Wasserstoff oder ein Rest ausgewählt aus der Gruppe bestehend aus einer gegebenenfalls substituierten Aryl-, Heteroaryl- und C₃-C₈-CycloalkylGruppe,
   und
n 0, 1, 2, 3, 4 oder 5,
bedeuten.

Insbesondere bevorzugt sind Verbindungen der Formel (I), worin
Q¹, Q², n, R⁴ bis R⁹ die oben genannten Bedeutungen aufweisen,
R¹, R² gleich oder verschieden, Wasserstoff oder ein Rest ausgewählt aus der Gruppe bestehend Methyl, Ethyl, Propyl , Allyl und Propargyl
   oder
R¹ und R² gemeinsam Cyclopropyl ,
R³ Wasserstoff, oder gegebenenfalls substituiertes C₁-C₆-Alkyl oder gegebenenfalls substituiertes und/oder verbrücktes C₃-C₁₂-Cycloalkyl,und
R¹⁰ und R¹¹ Wasserstoff bedeuten.
bedeuten.

Überaus bevorzugt sind Verbindungen der Formel (I), worin
Q¹, Q², n, R¹ bis R⁴, R⁶ bis R⁸, R¹⁰ und R¹¹ die oben genannten Bedeutungen aufweisen,
   und
R⁹ gleich oder verschieden, Wasserstoff oder einen Rest ausgewählt aus der Gruppe bestehend aus Halogen, (C₁-C₆-Alkyl)₂N-, CF₃, NH₂SO₂-, -CONH-C₆-C₁₄-Aryl, -CONH- C₁-C₄-alkyl-C₆-C₁₄-Aryl und -O-C₁-C₄-Alkyl ,
bedeuten.

Ein weiterer Gegenstand der Erfindung sind Verbindungen der Formel (I) zur Verwendung als Arzneimittel.

Erfindungsgemäß von besonderer Bedeutung sind Verbindung der Formel (I) zur Verwendung als Arzneimittel mit antiproliferativer Wirkung.

Ein weiterer Gegenstand der Erfindung ist die Verwendung einer Verbindung der Formel (I) zur Herstellung eines Arzneimittels zur Behandlung und/oder Prävention von Erkrankungen ausgewählt aus der Gruppe bestehend aus Krebs, bakteriellen und viralen Infektionen, Entzündungs- und Autoimmunerkrankungen, Chemotherapeutika induzierter Alopezie und Mukositis, kardiovaskulärer Erkrankungen, nephrologischen Erkrankungen, sowie chronisch und akut neurodegenerativen Erkrankungen, vorzugsweise zur Behandlung von Krebs, Entzündungs- und Autoimmunerkrankungen, insbesondere bevorzugt zur Behandlung von Krebs Und Entzündungserkrankungen.

Ein weiterer Gegenstand der Erfindung ist die Verwendung einer Verbindung der Formel (I) zur Herstellung eines Arzneimittels zur Inhibitierung der Polo-like Kinasen, insbesondere der Polo-like Kinase PLK-1.

Ein weiterer Gegenstand der Erfindung ist die Verwendung einer Verbindung der Formel (I) zur Herstellung eines Arzneimittels zur Behandlung und/oder Prävention von auf Überexpression der Polo-like Kinasen, insbesondere der PLK-1 Kinasen, beruhenden Tumorerkrankungen.

Ein weiterer Gegenstand der Erfindung ist eine Methode zur Behandlung und/oder Prävention von Erkrankungen ausgewählt aus der Gruppe bestehend aus Krebs, bakteriellen und viralen Infektionen, Entzündungs- und Autoimmunerkrankungen, Chemotherapeutika induzierter Alopezie und Mukositis, kardiovaskulärer Erkrankungen, nephrologischen Erkrankungen, sowie chronisch und akut neurodegenerativen Erkrankungen, vorzugsweise zur Behandlung von Krebs, Entzündungs-.und Autoimmunerkrankungen, insbesondere bevorzugt zur Behandlung von Krebs Und Entzündungserkrankungen, wobei man einem Patienten eine effektive Menge einer Verbindung der Formel (I) verabreicht.

Ein weiterer Gegenstand der Erfindung sind pharmazeutische Zubereitungen, enthaltend als Wirkstoff eine oder mehrere Verbindungen der allgemeinen Formel (I) gegebenenfalls in Kombination mit üblichen Hilfs- und/oder Trägerstoffen.

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 bis 6, besonders bevorzugt 1 bis 4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.
In den vorstehend genannten Alkylgruppen können gegebenenfalls ein oder mehrere Wasserstoffatome durch andere Reste ersetzt sein. Beispielsweise können diese Alkylgruppen durch Methyl, Chlor oder Fluor, vorzugsweise Fluor, substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 2 bis 5 Kohlenstoffatomen, beispielsweise Etyhlen, Propylen- , Isopropylen-, n-Butylen, iso-Butyl, sec. Butyl und tert.-Butyl etc. Brücken bezeichnet. Besonders bevorzugt sind Etyhlen, Propylen- und Butylen-Brücken. In den genannten Alkylbrücken können gegebenenfalls 1 bis 2 C-Atome durch ein oder mehrere Heteroatome ausgewählt aus der Gruppe Sauerstoff, Stickstoff oder Schwefel ersetzt sein.

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 1-Methyl-1-Propenyl, 1-Methyl-2-Propenyl, 2-Methyl-1-Propenyl, 2-Methyl-2-Propenyl und 1-Ethyl-1-Ethenyl.
In den vorstehend genannten Alkenylgruppen können, soweit nicht anders beschrieben, gegebenenfalls ein oder mehrere Wasserstoffatome durch andere Reste ersetzt sein. Beispielsweise können diese Alkenylgruppen durch Methyl, Chlor oder Fluor, vorzugsweise Fluor, substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkinylgruppen durch Methyl, Chlor oder Fluor, vorzugsweise Fluor, substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, OMe, -OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, - COOH, -COO-C₁-C₄-Alkyl, vorzugsweise -O-Methyl oder -O-Ethyl, oder -CONH₂.
Als Heteroarylreste, in denen bis zu zwei C-Atome durch ein oder zwei Stickstoffatome ersetzt sind werden beispielsweise, Pyrrol, Pyrazol, Imidazol, Triazol, Pyridin, Pyrimidin,, genannt, wobei jeder der vorstehend genannten Heteroarylringe gegebenenfalls ferner an einen Benzolring anneliert sein kann, vorzugsweise Benzimidazol, und wobei diese Heterocyclen ,soweit nicht anders beschrieben, beispielsweise einen oder mehrere der nachfolgend genannten Substituenten tragen können: F, Cl, Br, OH, OMe, Methyl, Ethyl, CN, CONH₂, NH₂,
gegebenenfalls substituiertes Phenyl, gegebenenfalls substituiertes Heteroaryl, vorzugsweise gegebenenfalls substituiertes Pyridyl.

Als Cycloalkylreste werden Cycloalkylreste mit 3 - 12 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen kann, beispielsweise: OH, NO₂, CN, OMe, -OCHF₂, -OCF₃, -NH₂ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -COO-Methyl oder -COO-Ethyl oder -CONH₂. Besonders bevorzugte Substituenten der Cycloalkylreste sind =O, OH, NH₂, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopententyl oder Cyclohexenyl bezeichnet, wobei jeder der vorstehend genannten Cycloalkenylreste gegebenenfalls ferner einen oder mehrere Substituenten tragen kann. "=O" bedeutet ein über eine Doppelbindung verknüpftes Sauerstoffatom.

Als Heterocycloalkylreste werden, soweit in den Definitionen nicht anders beschrieben, 3 bis 12 gliedrige, vorzugsweise 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Morpholin, Pyrrolidin, Piperidin oder Piperazin, genannt, wobei der Heterocyclus gegebenenfalls Substituenten tragen kann , beispielsweise C1-C4-Alkyl, vorzugsweise.Methyl, Ethyl oder Propyl.

Als Polycycloalkylreste werden gegebenenfalls substituierte, Bi-, Tri-tetra- oder pentacyclische Cycloalkylreste, beispielsweise Pinan, 2,2,2-Octan, 2,2,1-Heptan oder Adamantan, bezeichnet. Als Polycycloalkenylreste werden gegebenenfalls verbrückte oder/und substituierte, 8- gliedrige Bi-, Tri-tetra- oder pentacyclische Cycloalkenyllreste, vorzugsweise Bicycloalkenyl- oder Tricycloalkenylreste, sofern sie mindestens eine Doppelbindung aufweisen, beispielsweise .Norbornen, bezeichnet.
Als Spiroalkylreste werden gegebenenfalls substituierte spirocyclische C₅-C₁₂ Alkylreste bezeichnet.

Als Halogen wird im allgemeinen Fluor, Chlor, Brom oder Jod, vorzugsweise Fluor, Chlor oder Brom, besonders bevorzugt Chlor, bezeichnet.

Die erfindungsgemäßen Verbindungen können in Form der einzelnen optischen Isomeren, Mischungen der einzelnen Enantiomeren, Diastereomeren oder Racemate, in Form der Tautomere, in Form ihrer Solvate, vorzugsweise in Form ihrer Hydrate, sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

Der Substituent R¹ kann ein Rest ausgewählt aus der Gruppe bestehend aus gegebenenfalls substituiertem C₁-C₁₀-Alkyl, vorzugsweise C₁-C₄-Alkyl, besonders bevorzugt Methyl, Ethyl oder Propyl, C₂-C₁₀-Alkenyl, vorzugsweise Allyl, C₂-C₁₀-Alkinyl, vorzugsweise Propargyl, Aryl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁸-Aryl, -NR⁸-Heteroaryl, -NR⁸-Cycloalkyl, und -NR⁸-Heterocycloalkyl,
oder ein Rest ausgewählt aus der Gruppe bestehend aus Wasserstoff,
Halogen, COXR⁸, CON(R⁸)₂, COR⁸ und XR⁸, vorzugsweise Wasserstoff, bedeuten.
Vorzugsweise bedeutet der Substituent R¹ Ethyl oder Wasserstoff, besonders bevorzugt Wasserstoff.
Der Substituent R² kann ein Rest ausgewählt aus der Gruppe bestehend aus gegebenenfalls substituiertem C₁-C₁₀-Alkyl, vorzugsweise C₁-C₄-Alkyl, besonders bevorzugt Methyl, Ethyl oder Propyl, C₂-C₁₀-Alkenyl, vorzugsweise Allyl, C₂-C₁₀-Alkinyl, vorzugsweise Propargyl, Aryl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁸-Aryl, -NR⁸-Heteroaryl, -NR⁸-Cycloalkyl, und -NR⁸-Heterocycloalkyl,
oder ein Rest ausgewählt aus der Gruppe bestehend aus Wasserstoff, Halogen, COXR⁸, CON(R⁸)₂, COR⁸ und XR⁸, vorzugsweise Wasserstoff, bedeuten.
Vorzugsweise bedeutet der Substituent R² Ethyl oder Wasserstoff, besonders bevorzugt Ethyl.
Die Substituenten R¹ und R² können gemeinsam eine 2- bis 5-gliedrige Alkylbrücke, vorzugsweise eine 2-gliedrige Alkylbrücke, die 1 bis 2 Heteroatome, beispielsweise Sauerstoff, Schwefel oder Stickstoff, vorzugsweise Sauerstoff oder Stickstoff, enthalten kann, bedeuten.
Der Substituent R³ kann Wasserstoff oder ein Rest ausgewählt aus der Gruppe bestehend aus gegebenenfalls substituiertem C₁-C₁₂-Alkyl, vorzugsweise C₁-C₆-Alkyl, C₂-C₁₂-Alkenyl, C₂-C₁₂-Alkinyl, Aryl, Heteroaryl , -C₃-C₁₂-Cycloalkyl,
C₃-C₁₂-Cycloalkenyl, C₇-C₁₂-Polycycloalkyl, C₇-C₁₂-Polycycloalkenyl und C₅-C₁₂-Spirocycloalkyl oder
R¹ und R³ oder R² und R³ gemeinsam eine gesättigte oder ungesättigte C₃-C₄-Alkylbrücke, die 1 bis 2 Heteroatome enthalten kann,
Vorzugsweise bedeutet der Substituent R₃ C₁-C₆-Alkyl oder -C₃-C₁₂-Cycloalkyl, insbesondere bevorzugt Methyl oder Cyclopentyl.

Der Substituent R⁴ kann gegebenenfalls substituiertes Aryl oder Heteroaryl,vorzugsweise ein Rest der allgemeinen Formel 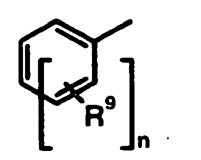 bedeuten.
Der Index n kann 0, 1, 2, 3, 4 oder 5, vorzugsweise 1 oder 2, besonders bevorzugt 1 bedeuten.
Der Substituent R⁵ kann ein Rest ausgewählt aus der Gruppe bestehend aus Wasserstoff, Halogen, CHO, XH, -X-C₁-C₂-Alkyl und einer gegebenenfalls substituierten C₁-C₃-Alkyl-Gruppe, vorzugsweise Methyl bedeuten. Vorzugsweise bedeutet der Substituent R⁵ Methyl.
Der Substituent R⁶ kann ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,bedeuten. Vorzugsweise bedeutet der Substituent R⁶ Wasserstoff.
Der Substituent R⁷ kann Wasserstoff,-CO-X-C₁-C₄-Alkyl, vorzugsweise -CO-O-Methyl oder -CO-O-Ethyl, -CO-NH-C₁-C₄-Alkyl, vorzugsweise -CO-NH-Methyl oder -CO-NH-Ethyl, oder -CO-C₁-C₄-Alkyl, vorzugsweise -CO-Methyl bedeuten. Vorzugsweise bedeutet der Substituent R⁷ Wasserstoff.
X kann jeweils unabhängig voneinander, Sauerstoff oder Schwefel, vorzugsweise Sauerstoff, bedeuten.
Der Substituent R⁸ kann jeweils unabhängig voneinander, Wasserstoff oder ein Rest ausgewählt aus der Gruppe bestehend aus gegebenenfalls substituiertem C₁-C₄-Alkyl, vorzugsweise Methyl oder Ethyl, C₂-C₄-Alkenyl, C₂-C₄-Alkinyl, Benzyl und Phenyl, bedeuten.
Vorzugsweise bedeutet der Substituent R⁸ Methyl, Phenyl oder Benzyl
Der Substituent R⁹ kann gleich oder verschieden, ein Rest ausgewählt aus der Gruppe bestehend aus gegebenenfalls substituiertem C₁-C₆-Alkyl, vorzugsweise Methyl, Ethyl oder Isopropyl,C₂-C₆-Alkenyl, vorzugsweise Allyl oder Homoallyl ,C₂-C₆-Alkinyl,vorzugsweise Propargyl -O-C₁-C₆-Alkyl, vorzugsweise -O-Methyl. oder O-Ethyl,-O-C₂-C₆-Alkenyl, -O-C₂-C₆-Alkinyl, C₃-C₆-Heterocycloalkyl, vorzugsweise Piperazinyl, Morpholinyl, Pyrrolidinyl oder Piperidinyl,C₃-C₆-Cycloalkyl, vorzugsweise Cyclopropyl, Cyclopentyl oder Cyclohexyl,Aryl, vorzugsweise Phenyl, oder Naphthalinyl Heteroaryl, vorzugsweise Pyridinyl oder Pyrimidinyl, -O-Aryl, vorzugsweise -O-Phenyl.O-Heteroaryl, vorzugsweise o- Pyridinyl oder O-Pyrimidinyl, -O-Cycloalkyl, vorzugsweise O-Cyclopentyl. oder O-Cyclohexyl, und -O-Heterocycloalkyl vorzugsweise -O-Morpholinyl, -O- Piperidinyl, -O-Pyrrolidinyl, oder -O-Piperazinyl, oder
ein Rest ausgewählt aus der Gruppe bestehend aus Wasserstoff, -CONH₂, - COOR⁸, -OCON(R⁸)₂, -N(R⁸)₂, -NHCOR⁸, -NHCON(R⁸)₂, -NO₂, CF₃, Halogen, - O-C₁-C₆-Alkyl-Q¹, -CONR⁸-C₁-C₁₀-Alkyl-Q¹, -CONR⁸-C₂-C₁₀-Alkenyl-Q¹, -CONR⁸-Q², Halogen, OH, -SO₂R⁸, -SO₂N(R⁸)₂, -COR⁸, -COOR⁸, -N(R⁸)₂, - NHCOR⁸, -CONR⁸OC₁-C₁₀ -Alkyl-Q¹ und -CONR⁸O-Q²,
Vorzugsweise bedeutet der Substituent R⁹ -CONR⁸-Q² oder -CONR⁸OC₁-C₁₀ - Alkyl-Q¹ .
Der Substituenten R¹⁰ und R¹¹ können jeweils unabhängig voneinander, Wasserstoff oder gegebenenfalls substituiertes C₁-C₄-Alkyl , vorzugsweise Wasserstoff oder Methyl, oder
R¹⁰ und R¹¹ können gemeinsam eine 2-5 gliedrige Alkylbrücke, vorzugsweise eine 2-gliedrige Alkylbrücke, bedeuten.
Besonders bevorzugt bedeuten die Substituenten R¹⁰ und R¹¹ Wasserstoff.

Q¹ kann Wasserstoff, -NHCOR⁸ oder ein Rest ausgewählt aus der Gruppe bestehend aus einer gegebenenfalls substituierten -NH-Aryl-, -NH-Heteroaryl-, Aryl-, vorzugsweise Phenyl, Heteroaryl-, C₃-C₈-Cycloalkyl- und Heterocycloalkyl-Gruppe, bedeuten.
Q² kann Wasserstoff oder ein Rest ausgewählt aus der Gruppe bestehend aus einer gegebenenfalls substituierten Aryl-, vorzugsweise Phenyl, Heteroaryl- und C₃-C₈-Cycloalkyl-Gruppe, bedeuten.

Alle in der Bedeutung für R¹ bis R⁹ genannten Reste können gegebenenfalls verzweigt und/oder substituiert sein.

Die Verbindungen der allgemeinen Formel (1) können nach folgendem Syntheseverfahren hergestellt werden, wobei die Substituenten der allgemeinen Formeln (A1), (A2) und (I) die zuvor genannten Bedeutungen haben. Dieses Verfahren ist als Erläuterung der Erfindung zu verstehen ohne selbige auf deren Gegenstand zu beschränken.

Eine Verbindung der Formel (A1) wird mit einem gegebenfalls substituiertem Benzylamin (A2) zu der allgemeinen Formel (I) umgesetzt. Die Verbindungen der Formel (A1) können wie auf Seite 23 der WO 2003020722 beschrieben erhalten werden. Analog zu dem dort beschriebenen Verfahren können auch enantiomerenreine Verbinungen (A1) bezüglich der Stereochemie an R1/R2 erhalten werden. Das verwendete 4-Aminomethyl-N-benzylbenzamid kann z.B. nach folgender Literaturstelle erhalten werden: H.G. Kazmirowski, P. Neuland, H. Landmann, F. Markwardt Pharmazie 1967, 22(9), 465-70.

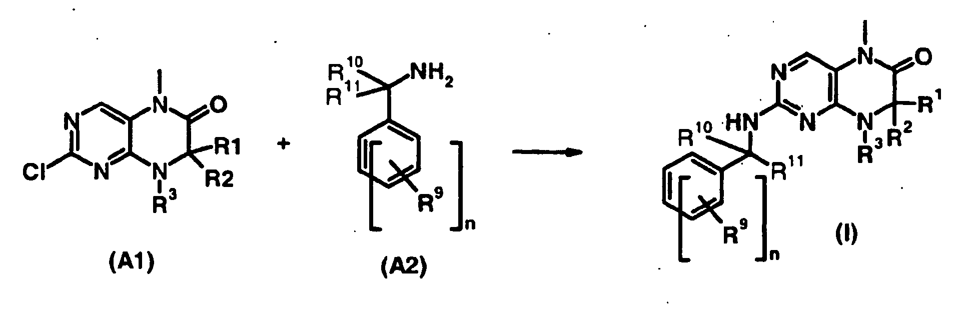

Die neuen Verbindungen der allgemeinen Formel (I) können in Analogie der nachfolgenden Beispiele dargestellt werden.

Folgende 2-Chlor-dihydropteridinone wurden in den Synthesen eingesetzt:

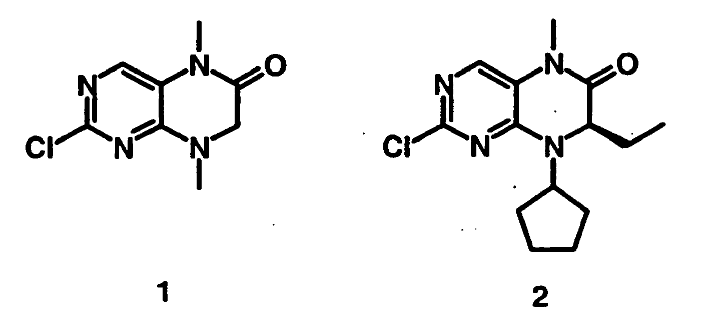

### Beispiel 9

0,5 g 1 wurde zusammen mit 1,15 g 4-Aminomethyl-N-benzylbenzamid in 2,5 mL Sulfolan für 30 min auf 160°C gerührt. Nach dem Abkühlen der Reaktionslösung wurde mit 20 mL Methanol verdünnt und der gebildete Niederschlag abfiltriert, mit Methanol nachgewaschen und getrocknet. Es ergab 470 mg hellbraune Kristalle mit Smp. 162°C.

### Beispiel 10

0,2 g 1 wurde mit 0,4 g 4-Arninomethylbenzolsulfonamid x HCl und 0,34 mL 30%iger Natriummethylatlösung in 1 mL Sulfolan bei 160°C für 2 h gerührt.

Nach dem Abkühlen wurde mit Methanol und Ether verdünnt, vom Niederschlag abfiltriert, die Mutterlauge eingeengt und der Rückstand mit Methylenchlorid und 2N Salzsäure extrahiert. Aus der Methylenchloridphase kristallisiert ein gelber Niederschlag aus, der abfiltriert und getrocknet wurde. Es ergab 40 mg gelbe Kristalle mit Smp. 248°C.

### Beispiel 11

0,1 g 2 wurde zusammen mit 168 µL Benzylamin ohne Lösungsmittel bei 160°C für 1 h und 45 Minuten zusammengeschmolzen. Der Rückstand wurde nach dem Abkühlen mit wenig Dichlormethan und Methanol gelöst und mit Ether versetzt. Der gebildete Niederschlag wurde abfiltriert und die Mutterlauge eingeengt und einer Kieselgelchromatographie unterworfen. Als Eluent wurde Dichlormethan: Methanol : wässrige Ammoniaklösung im Verhältnis 95 : 5 : 0,5 angewendet. Anschließend wurden die produktenthaltenden Fraktionen vereinigt und eingeengt. Der Rückstand wurde in Ethylacetat gelöst, mit etherischer HCl und Petrolether versetzt und der Niederschlag abfiltriert. Es ergab 0,08 g eines hellbraunen hygroskopischen Feststoffes.

### Beispiel 12

0,1 g 2 und 95 µL 3-Chlorbenzylamin wurden ohne Lösungsmittel 2h bei 160°C erhitzt. Die abgekühlte Reaktionsmischung wurde mit Methanol und Dichlormethan digeriert und mit Ether versetzt. Der Niederschlag wurde abfiltriert und die Mutterlauge chromatographisch wie in Beispiel 11 beschrieben gereinigt. Die Produktfraktionen wurden vereinigt und eingeengt. Anschließend wurde das Produkt in Aceton gelöst, mit einer Oxalsäurelösung in Isopropanol versetzt und der Niederschlag abfiltriert und getrocknet. Es ergab 72 mg eines weißen Feststoffes mit Smp. 171°C.

### Beispiel 14

0,1 g 2 und 100 µL 3-Methoxybenzylamin wurden ohne Lösungsmittel 2h bei 160°C erhitzt. Die abgekühlte Reaktionsmischung wurde mit Methanol und Dichlormethan digeriert und mit Ether versetzt. Der Niederschlag wurde abfiltriert und die Mutterlauge chromatographisch wie in Beispiel 11 beschrieben gereinigt. Die Produktfraktionen wurden vereinigt und eingeengt. Anschließend wurde das Produkt in Aceton gelöst, mit einer Oxalsäurelösung in Isopropanol versetzt und der Niederschlag abfiltriert und getrocknet. Es ergab 97 mg eines weißen Feststoffes mit Smp. 175°C.

Analog zu vorstehend beschriebener Vorgehensweise wurden u.a. die in Tabelle 1 aufgeführten Verbindungen der allgemeinen Formel (I) erhalten.

**Tabelle 1**

| | | | | | | | |
|---|---|---|---|---|---|---|---|
| 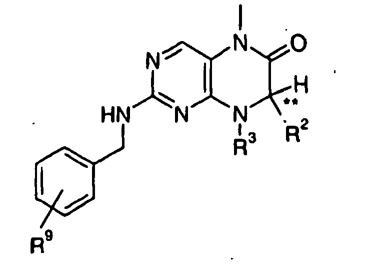 | | | | | | | |

| Beispiel | Konfig. **H/R2 | R² | R³ | R⁹ ortho | R⁹ meta | R⁹ para | Smp. |
|---|---|---|---|---|---|---|---|
| 1 | | H | -CH₃ | H | H | H | |
| 2 | | H | -CH₃ | H | H | Cl | 250°C |
| 3 | | H | -CH₃ | H | -CF₃ | H | 206°C |
| 4 | | H | -CH₃ | H | -OCH₃ | H | 194°C |
| 5 | | H | -CH₃ | H | H | -OCH₃ | 195°C |
| 6 | | H | -CH₃ | H | H | F | 253°C |
| 7 | | H | -CH₃ | -OCH₃ | H | H | 194°C |
| 8 | | H | CH₃ | H | H | -N(CH₃)₂ | 235°C |
| 9 | | H | -CH₃ | H | H | 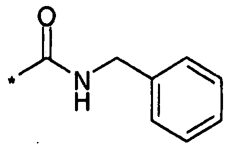 | 162°C |
| 10 | | H | -CH₃ | H | H | -SO₂NH₂ | 248°C |
| 12 | R | -CH₂CH₃ | 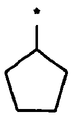 | H | Cl | H | 171°C |
| 13 | R | -CH₂CH₃ | 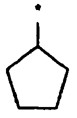 | H | H | Cl | 149°C |
| 14 | R | -CH₂CH₃ | 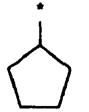 | -OCH₃ | H | H | 175°C |
| 15 | R | -CH₂CH₃ | 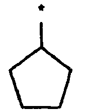 | H | H | H | 144°C |
| 16 | R | -CH₂CH₃ | 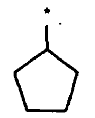 | Cl | H | H | 177°C |
| 17 | R | -CH₂CH₃ | 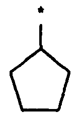 | H | H | -OCH₃ | |

| | | | | | | | |
|---|---|---|---|---|---|---|---|
| * Bindungsposition | | | | | | | |

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch DNA-Färbung mit darauf folgender FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001). Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, Hormone oder Antikörper verwendet werden.

Die Wirkung der erfindungsgemäßen Verbindungen wurde im PLK1 Inhibitionsassay, im Cytotoxizitätstest an kultivierten humanen Tumorzellen und/oder in einer FACS-Analyse, beispielsweise an HeLa 53-Zellen, bestimmt. Die Verbindungen zeigten in beiden Testmethoden eine gute bis sehr gute Wirksamkeit, d.h. beispielsweise einen EC₅₀-Wert im HeLa S3-Cytotoxizitätstest kleiner 5 µmol/L, in der Regel kleiner 1 µmol/L und einen IC₅₀-Wert im PLK1-Inhibitionsassay kleiner 1 µmol/L.

### PLK1 Kinaseassay

### Enzymherstellung:

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät. Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/I, 0,2 g KCl/l, 1,44 g Na₂HPO₄/l, 0,24 g KH₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM β-Glycerolphosphat, 0.1 mM Na₃VO₄, 30 mM 4-Nitrophenylphosphate) zu 1x10⁸ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCl pH=8,0, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt.

### Assaydurchführung:

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration (z.B. beginnend bei 300 µM, und Verdünnung in 1:3) in 6% DMSO, 0,5 mg/ml Casein (Sigma C-5890), 60 mM β-Glycerophosphat, 25 mM MOPS pH=7,0, 5 mM EGTA, 15 mM MgCl₂, 1 mM DTT
- 20 µl Substratlösung (25 mM MOPS pH=7,0, 15 mM MgCl₂, 1 mM DTT, 2,5 mM EGTA, 30 mM β-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35µl Szintillationslösung (Ready-Safe; Beckmann) pro Napf wird die Platte mit Sealing-tape zugeklebt und die präzipitierte Menge P33 mit dem Wallac Betacounter gemessen.
Die Messdaten werden mit der Standard Graphpad Software (Levenburg-Marquard Algorhythmus) ausgewertet.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLa S3 Zellen in 96-well Platten (Costar) mit einer Dichte von 1000 Zellen pro well eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue Reagenz). Die Wirksubstanzen wurden in verschiedenen Konzentrationen (gelöst in DMSO; DMSO-Endkonzentration: 0.1 %) zu den Zellen zugegeben (jeweils als Dreifachbestimmung). Nach 72 Stunden Inkubation wurden zu jeden well 20 µl AlamarBlue Reagenz(AccuMed International) zugesetzt, und die Zellen für weitere 7 Stunden inkubiert. Zur Kontrolle wurde zu 3 wells je 20 µl reduziertes AlamarBlue Reagenz gegeben (AlamarBlue Reagenz, das für 30 min autoklaviert wurde). Nach 7 h Inkubation wurde der Farbumsatz des AlamarBlue Reagenz in den einzelnen wells in einem Perkin Eimer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (HeLa S3 Zellen ohne Inhibitor) berechnet und die Wirkstoffkonzentration, die die Zellaktivität zu 50% hemmt (IC₅₀) abgeleitet. Die Werte wurden hierbei aus dem Mittelwert von drei Einzelbestimmungen - unter Korrektur des Leerwertes (Mediumkontrolle)- berechnet.

### FACS- Analyse

Propidium lodid (P1) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N) , während Zellen in der G2 oder Mitose einen 4N DNA Gehalt haben. Für eine PI-Färbung wurden beispielsweise 0.4 Mio HeLa S3 Zellen auf eine 75 cm² Zellkulturflasche ausgesät, nach 24 h wurde entweder 0.1 % DMSO als Kontrolle zugesetzt, bzw. die Substanz in verschiedenen Konzentrationen (in 0.1 % DMSO). Die Zellen wurden für 24 h mit der Substanz bzw. mit DMSO inkubiert, bevor die Zellen 2 x mit PBS gewaschen und dann mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C) , mit PBS gewaschen und anschließend nochmals zentrifugiert . Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl PI Färbelösung (0.1 mg/ml RNase A, 10 µg/ml Prodium lodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische PI Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend, wurden erfindungsgemäße Verbindungen auf weiteren Tumorzellen getestet. Beispielsweise sind diese Verbindungen auf Karzinomen verschiedenster Gewebe (z. Bspl. Brust (MCF7); Colon (HCT116), Kopf-Hals (FaDu), Lunge (NCI-H460), Pankreas (BxPC-3), Prostata (DU145)), Sarkome (z. Bspl. SK-UT-1 B), Leukämien und Lymphome (z. Bspl. HL-60; Jurkat, THP-1) und anderen Tumoren (z. Bspl. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.
Für den Fall der parenteralen Anwendung können Lösungen der Wirkstoffe unter Verwendung geeigneter flüssiger Trägermaterialien eingesetzt werden. Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feuchtgranuliert und getrocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als lsotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I), 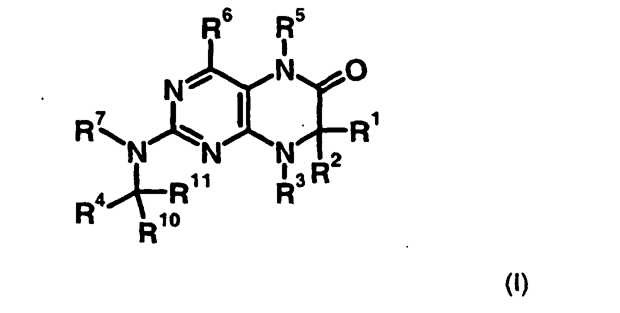 worin
R¹, R² gleich oder verschieden, ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl,
C₃-C₈-Heterocycloalkyl, -X-Aryl, -X-Heteroaryl, -X-Cycloalkyl, -X-Heterocycloalkyl, -NR⁸-Aryl, -NR⁸-Heteroaryl, -NR⁸-Cycloalkyl und -NR⁸-Heterocycloalkyl,
oder
ein Rest ausgewählt aus der Gruppe bestehend aus Wasserstoff, Halogen, COXR⁸, CON(R⁸)₂, COR⁸ und XR⁸,
oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl, Aryl, Heteroaryl , -C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl oder R¹ und R³ oder R² und R³ gemeinsam eine gesättigte oder ungesättigte C₃-C₄-Alkylbrücke, die 1 bis 2 Heteroatome enthalten kann,
R⁴ gegebenenfalls substituiertes Aryl oder Heteroaryl;
R⁵ ein Rest ausgewählt aus der Gruppe bestehend aus Wasserstoff, CHO, XH, -X-C₁-C₂-Alkyl und einer gegebenenfalls substituierten C₁-C₃-AlkylGruppe,
R⁶ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
R⁷ Wasserstoff, -CO-NH-C₁-C₄-Alkyl, -CO-C₁-C₄-Alkyl oder -CO-X-C₁-C₄-Alkyl, und
X jeweils unabhängig voneinander, O oder S,
R⁸ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl, Benzyl und Phenyl,
R¹⁰ und R¹¹ jeweils unabhängig voneinander, Wasserstoff oder gegebenenfalls substituiertes C₁-C₄-Alkyl,
oder R¹⁰ und R¹¹ gemeinsam eine 2-5 gliedrige Alkylbrücke,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate, bedeuten.

2. Verbindungen nach Anspruch 1,
worin
R¹ bis R⁴ die angegebene Bedeutung aufweisen und
R⁵ Methyl,
R⁶ und R⁷ Wasserstoff, und
R¹⁰ und R¹¹_{,}jeweils unabhängig voneinander, Wasserstoff oder Methyl,
oder R¹⁰ und R¹¹ gemeinsam Cyclopropyl, bedeuten.

3. Verbindungen nach Anspruch 1 oder 2,
worin
R³ bis R⁷, R¹⁰ und R¹¹ die angegebene Bedeutung aufweisen und
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
oder
R¹ und R² gemeinsam eine 2- bis 5-gliedrige Alkylbrücke,
bedeuten.

4. Verbindungen nach einem der Ansprüche 1 bis 3,
worin
R¹, R³ bis R⁷ ,R¹⁰ und R¹¹ die angegebene Bedeutung aufweisen,
und
R³ Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl, bedeutet.

5. Verbindungen nach einem der Ansprüche 1 bis 4,
worin
R¹ bis R³ , R⁵ bis R⁷, R¹⁰ und R¹¹ die angegebene Bedeutung aufweisen,
und
R⁴ ein Rest der allgemeinen Formel 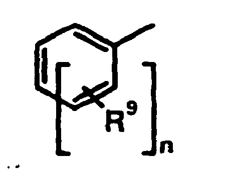 worin
R⁹ gleich oder verschieden, ein Rest ausgewählt aus der Gruppe bestehend aus gegebenenfalls substituiertem C₁-C₆-Alkyl, C₂-C₆-Alkenyl, C₂-C₆-Alkinyl, -O-C₁-C₆-Alkyl, -O-C₂-C₆-Alkenyl, -O-C₂-C₆-Alkinyl, C₃-C₆-Heterocycloalkyl, C₃-C₆-Cycloalkyl, Aryl, Heteroaryl, -O-Aryl, -O-Heteroaryl, -O-Cycloalkyl, und -O-Heterocycloalkyl oder
ein Rest ausgewählt aus der Gruppe bestehend aus Wasserstoff, -CONH₂, - COOR⁸_{,}-OCON(R⁸)₂, -N(R⁸)₂, -NHCOR⁸_{,}-NHCON(R⁸)₂, -NO₂, CF₃, Halogen, - O-C₁-C₆-Alkyl-Q¹, -CONR⁸-C₁-C₁₀-Alkyl-Q¹, -CONR⁸-C₂-C₁₀-Alkenyl-Q¹, -CONR⁸-Q², OH, -SO₂R⁸, -SO₂N(R⁸)₂, -COR⁸, -COOR⁸, -CONR⁸OC₁-C₁₀ -Alkyl-Q¹ und CONR⁸O-Q²,
R⁸ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl und Phenyl,
Q¹ Wasserstoff, -NHCOR⁸, oder ein Rest ausgewählt aus der Gruppe bestehend aus einer gegebenenfalls substituierten -NH-Aryl-, -NH-Heteroaryl-, Aryl-, Heteroaryl-, C₃-C₈-Cycloalkyl- und Heterocycloalkyl-Gruppe,
Q² Wasserstoff oder ein Rest ausgewählt aus der Gruppe bestehend aus einer gegebenenfalls substituierten Aryl-, Heteroaryl- und C₃-C₈-CycloalkylGruppe,
und
n 0, 1, 2, 3, 4 oder 5,
bedeuten.

6. Verbindungen nach einem der Ansprüche 1 bis 5,
worin
Q¹, Q², n, R⁴ bis R⁸ die angegebene Bedeutung aufweisen,
R¹, R² gleich oder verschieden, Wasserstoff oder ein Rest ausgewählt aus der Gruppe bestehend Methyl, Ethyl, Propyl , Allyl und Propargyl
oder
R¹ und R² gemeinsam Cyclopropyl,
R³ gleich Wasserstoff, oder gegebenenfalls substituiertes C₁-C₆-Alkyl oder gegebenenfalls substituiertes und/oder verbrücktes C₃-C₁₂-Cycloalkyl, und
R¹⁰ und R¹¹ Wasserstoff
bedeuten.

7. Verbindungen nach Anspruch 5 oder 6,
worin
Q¹, Q², n, R¹ bis R⁴, R⁶ bis R⁸, R¹⁰ und R¹¹ die angegebene Bedeutung aufweisen,
und
R⁹ gleich oder verschieden, Wasserstoff oder einen Rest ausgewählt aus der Gruppe bestehend aus Halogen, (C₁-C₄-Alkyl)₂N-, CF₃, NH₂SO₂-, -CONH-C₆-C₁₄-Aryl, -CONH- C₁-C₄-alkyl-C₆-C₁₄-Aryl und -O-C₁-C₄-Alkyl, bedeuten.

8. Verbindung der allgemeinen Formel (I) nach einem der Ansprüche 1 bis 7 zur Verwendung als Arzneimittel.

9. Verbindung der allgemeinen Formel (I) nach einem der Ansprüche 1 bis 7 zur Verwendung als Arzneimittel mit antiproliferativer Wirkung.

10. Verwendung einer Verbindung der allgemeinen Formel (I) gemäß einem der Ansprüche 1 bis 7 zur Herstellung eines Arzneimittels zur Behandlung und/oder Prävention von Erkrankungen ausgewählt aus der Gruppe bestehend aus Krebs, bakteriellen und viralen Infektionen, Entzündungs- und Autoimmunerkrankungen, Chemotherapeutika induzierter Alopezie und Mukositis, kardiovaskulärer Erkrankungen, nephrologischen Erkrankungen, sowie chronisch und akut neurodegenerativen Erkrankungen.

11. Verwendung einer Verbindung der allgemeinen Formel (I) gemäß einem der Ansprüche 1 bis 7 zur Herstellung eines Arzneimittels zur Inhibitierung der Polo-like Kinasen.

12. Verwendung gemäß Anspruch 11, **dadurch gekennzeichnet, dass** die Polo-like Kinase PLK1 ist.

13. Verwendung einer Verbindung der allgemeinen Formel (I) gemäß einem der Ansprüche 1 bis 7 zur Herstellung eines Arzneimittels zur Behandlung und/oder Prävention von auf Überexpression der Polo-like Kinasen, beruhenden Tumorerkrankungen.

14. Pharmazeutische Zubereitungen, enthaltend als Wirkstoff eine oder mehrere Verbindungen der allgemeinen Formel (I) gemäß einem der Ansprüche 1 bis 7 gegebenenfalls in Kombination mit üblichen Hilfs- und/oder Trägerstoffen.

## Claims

1. Compounds of general formula (I), 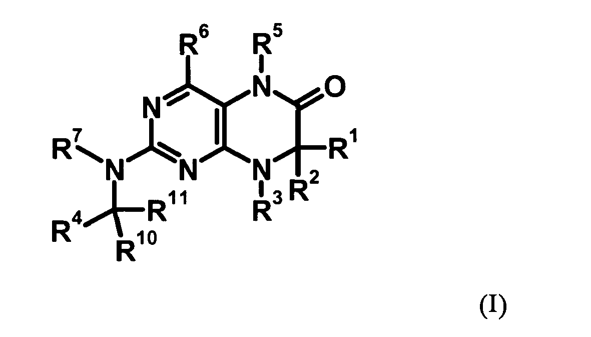 wherein
R¹, R² which may be identical or different, denote a group selected from the group consisting of optionally substituted C₁-C₁₀-alkyl, C₂-C₁₀-alkenyl, C₂-C₁₀-alkynyl, aryl, heteroaryl, C₃-C₈-cycloalkyl, C₃-C₈-heterocycloalkyl, -X-aryl, -X-heteroaryl, -X-cycloalkyl, -X-heterocycloalkyl, -NR⁸-aryl, -NR⁸-heteroaryl, - NR⁸-cycloalkyl, and -NR⁸-heterocycloalkyl,
or
a group selected from the group consisting of hydrogen, halogen, COXR⁸, CON(R⁸)₂, COR⁸ and XR⁸,
or
R¹ and R² together denote a 2- to 5-membered alkyl bridge which may contain 1 to 2 heteroatoms,
R³ denotes hydrogen or a group selected from the group consisting of optionally substituted C₁-C₁₂-alkyl, C₂-C₁₂-alkenyl, C₂-C₁₂-alkynyl, aryl, heteroaryl, -C₃-C₁₂-cycloalkyl, C₃-C₁₂-cycloalkenyl, C₇-C₁₂-polycycloalkyl, C₇-C₁₂-polycycloalkenyl and C₅-C₁₂-spirocycloalkyl or
R¹ and R³ or R² and R³ together denote a saturated or unsaturated C₃-C₄-alkyl bridge which may contain 1 to 2 heteroatoms,
R⁴ denotes optionally substituted aryl or heteroaryl,
R⁵ denotes a group selected from the group consisting of hydrogen, CHO, XH, -X-C₁-C₂-alkyl and an optionally substituted C₁-C₃-alkyl group,
R⁶ denotes a group selected from the group consisting of hydrogen, NH₂, XH, halogen and a C₁-C₃-alkyl group optionally substituted by one or more halogen atoms,
R⁷ denotes hydrogen, -CO-NH-C₁-C₄-alkyl, -CO-C₁-C₄-alkyl or -CO-X-C₁-C₄-alkyl, and
X in each case independently of one another denote O or S,
R⁸ in each case independently of one another denote hydrogen or a group selected from the group consisting of optionally substituted C₁-C₄-alkyl, C₂-C₄-alkenyl, C₂-C₄-alkynyl, benzyl and phenyl,
R¹⁰ and R¹¹ in each case independently of one another denote hydrogen or optionally substituted C₁-C₄-alkyl,
or R¹⁰ and R¹¹ together denote a 2-5 membered alkyl bridge,
optionally in the form of the tautomers, racemates, enantiomers, diastereomers and mixtures thereof, and optionally the pharmacologically acceptable acid addition salts, solvates or hydrates thereof.

2. Compounds according to claim 1,
wherein
R¹ to R⁴ have the meanings specified, and
R⁵ denotes methyl,
R⁶ and R⁷ denote hydrogen, and
R¹⁰ and R¹¹ in each case independently of one another denote hydrogen or methyl,
or
R¹⁰ and R¹¹ together denote cyclopropyl.

3. Compounds according to claim 1 or 2,
wherein
R³ to R⁷, R¹⁰ and R¹¹ have the meanings specified and
R¹, R² which may be identical or different, denote hydrogen or a group selected from the group consisting of optionally substituted C₁-C₆-alkyl, C₂-C₆-alkenyl, and C₂-C₆-alkynyl,
or
R¹ and R² together denote a 2- to 5-membered alkyl bridge.

4. Compounds according to one of claims 1 to 3,
wherein
R¹, R³ to R⁷, R¹⁰ and R¹¹ have the meanings specified,
and
R³ denotes hydrogen or a group selected from the group consisting of optionally substituted C₁-C₁₂-alkyl, C₂-C₁₂-alkenyl, C₂-C₁₂-alkynyl and C₆-C₁₄-aryl, or
a group selected from the group consisting of optionally substituted and/or bridged C₃-C₁₂-cycloalkyl, C₃-C₁₂-cycloalkenyl, C₇-C₁₂-polycycloalkyl, C₇-C₁₂-polycycloalkenyl and C₅-C₁₂-spirocycloalkyl.

5. Compounds according to one of claims 1 to 4,
wherein
R¹ to R³, R⁵ to R⁷, R¹⁰ and R¹¹ have the meanings specified,
and
R⁴ denotes a group of general formula 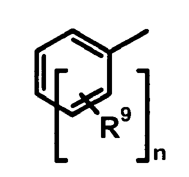 wherein
R⁹ which may be identical or different, denotes a group selected from the group consisting of optionally substituted C₁-C₆-alkyl, C₂-C₆-alkenyl, C₂-C₆-alkynyl, -O-C₁-C₆-alkyl, -O-C₂-C₆-alkenyl, -O-C₂-C₆-alkynyl, C₃-C₆-heterocycloalkyl, C₃-C₆-cycloalkyl, aryl, heteroaryl, -O-aryl, -O-heteroaryl, -O-cycloalkyl, and -O-heterocycloalkyl or
a group selected from the group consisting of hydrogen, -CONH₂, -COOR⁸, - OCON(R⁸)₂, -N(R⁸)₂, -NHCOR⁸, -NHCON(R⁸)₂, -NO₂, CF₃, halogen, -O-C₁-C₆-alkyl-Q¹, -CONR⁸-C₁-C₁₀-alkyl-Q¹, -CONR⁸-C₂-C₁₀-alkenyl-Q¹, -CONR⁸-Q², OH, -SO₂R⁸, -SO₂N(R⁸)₂, -COR⁸, -COOR⁸, -CONR⁸OC₁-C₁₀-alkyl-Q¹ and CONR⁸O-Q²,
R⁸ in each case independently of one another denote hydrogen or a group selected from the group consisting of optionally substituted C₁-C₄-alkyl, C₂-C₄-alkenyl, C₂-C₄-alkynyl and phenyl,
Q¹ denotes hydrogen, -NHCOR⁸, or a group selected from the group consisting of an optionally substituted -NH-aryl, -NH-heteroaryl, aryl, heteroaryl, C₃-C₈-cycloalkyl and heterocycloalkyl group,
Q² denotes hydrogen or a group selected from the group consisting of an optionally substituted aryl, heteroaryl and C₃-C₈-cycloalkyl group,
and
n denotes 0, 1, 2, 3, 4 or 5.

6. Compounds according to one of claims 1 to 5,
wherein
Q¹, Q², n, R⁴ to R⁸ have the meanings specified,
R¹, R², which may be identical or different, denote hydrogen or a group selected from the group consisting of methyl, ethyl, propyl, allyl and propargyl
or
R¹ and R² together represent cyclopropyl,
R³ denotes hydrogen, or denotes optionally substituted C₁-C₆-alkyl or optionally substituted and/or bridged C₃-C₁₂-cycloalkyl, and
R¹⁰ and R¹¹ denote hydrogen.

7. Compounds according to claim 5 or 6,
wherein
Q¹, Q², n, R¹ to R⁴, R⁶ to R⁸, R¹⁰ and R¹¹ have the meanings specified,
and
R⁹, which may be identical or different, denote hydrogen or a group selected from the group consisting of halogen, (C₁-C₄-alkyl)₂N-, CF₃, NH₂SO₂-, -CONH-C₆-C₁₄-aryl, -CONH-C₁-C₄-alkyl-C₆-C₁₄-aryl and -O-C₁-C₄-alkyl.

8. Compound of general formula (I) according to one of claims 1 to 7 for use as a pharmaceutical composition.

9. Compound of formula I according to one of claims 1 to 7 for use as a pharmaceutical composition with an antiproliferative activity.

10. Use of a compound of general formula (I) according to one of claims 1 to 7 for preparing a pharmaceutical composition for the treatment and/or prevention of diseases selected from the group consisting of cancer, bacterial and viral infections, inflammatory and autoimmune diseases, chemotherapy-induced alopecia and mucositis, cardiovascular diseases, nephrological diseases, as well as chronic and acute neurodegenerative diseases.

11. Use of a compound of general formula (I) according to one of claims 1 to 7 for preparing a pharmaceutical composition for inhibiting polo-like kinases.

12. Use according to claim 11, **characterised in that** the polo-like kinase is PLK1.

13. Use of a compound of general formula (I) according to one of claims 1 to 7 for preparing a pharmaceutical composition for the treatment and/or prevention of tumoral diseases based on overexpression of the polo-like kinases.

14. Pharmaceutical preparations, containing as active substance one or more compounds of general formula (I) according to one of claims 1 to 7 optionally combined with conventional excipients and/or carriers.

## Revendications

1. Composés de formule générale (I) 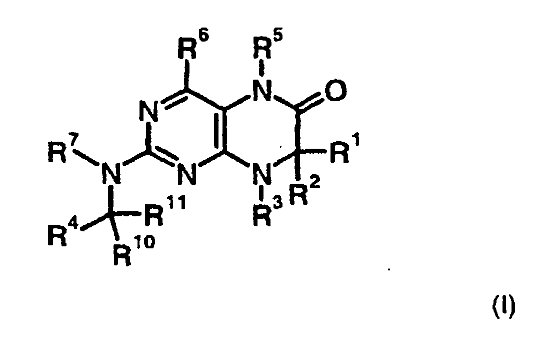 où
R¹, R², identiques ou différents, représentent un groupement choisi dans le groupe consistant en C₁-C₁₀-alkyle, C₂-C₁₀-alcényle, C₂-C₁₀-alcynyle, aryle, hétéroaryle, C₃-C₈-cycloalkyle, C₃-C₈-hétérocycloalkyle, -X-aryle, -X-hétéroaryle, -X-cycloalkyle, -X-hétérocycloalkyle, -NR⁸-aryle, -NR⁸-hétéroaryle, -NR⁸-cycloalkyle et -NR⁸-hétérocycloalkyle éventuellement substitué,
ou
un groupement choisi dans le groupe consistant en l'hydrogène, halogène, COXR⁸, CON(R⁸)₂, COR⁸ et XR⁸,
ou
R¹ et R² représentent ensemble un pont alkyle à 2 à 5 chaînons, qui peut contenir 1 à 2 hétéroatomes,
R³ représente l'hydrogène ou un groupement choisi dans le groupe consistant en C₁-C₁₂-alkyle, C₂-C₁₂-alcényle, C₂-C₁₂-alcynyle, aryle, hétéroaryle, -C₃-C₁₂-cycloalkyle, C₃-C₁₂-cycloalcényle, C₇-C₁₂-polycycloalkyle, C₇-C₁₂-polycycloalcényle et C₅-C₁₂-spirocycloalkyle éventuellement substitué ou
R¹ et R³ ou R² et R³ représentent ensemble un pont C₃-C₄-alkyle saturé ou insaturé, qui peut contenir 1 à 2 hétéroatomes,
R⁴ représente aryle ou hétéroaryle éventuellement substitué,
R⁵ représente un groupement choisi dans le groupe consistant en l'hydrogène, CHO, XH, -X-C₁-C₂-alkyle et un groupe C₁-C₃-alkyle éventuellement substitué,
R⁶ représente un groupement choisi dans le groupe consistant en l'hydrogène, NH₂, XH, halogène et un groupe C₁-C₃-alkyle éventuellement substitué par un ou plusieurs atomes d'halogène,
R⁷ représente l'hydrogène, -CO-NH-C₁-C₄-alkyle, -CO-C₁-C₄-alkyle ou -CO-X-C₁-C₄-alkyle, et
X représente dans chaque cas indépendamment O ou S ;
R⁸ représente dans chaque cas indépendamment l'hydrogène ou un groupement choisi dans le groupe consistant en C₁-C₄-alkyle, C₂-C₄-alcényle, C₂-C₄-alcynyle, benzyle et phényle éventuellement substitué,
R¹⁰ et R¹¹ représentent dans chaque cas indépendamment l'un de l'autre l'hydrogène ou C₁-C₄-alkyle éventuellement substitué,
ou R¹⁰ et R¹¹ représentent ensemble un pont alkyle à 2-5 chaînons,
éventuellement sous forme de leurs tautomères, de leurs racémates, de leurs énantiomères, de leurs diastéréoisomères et de leurs mélanges, ainsi que éventuellement sous forme de leurs sels d'addition d'acide, solvates ou hydrates pharmacologiquement acceptables.

2. Composés selon la revendication 1 où
R¹ à R⁴ présentent la signification indiquée et
R⁵ représente méthyle,
R⁶ et R⁷ représentent l'hydrogène et
R¹⁰ et R¹¹ représentent dans chaque cas indépendamment l'un de l'autre l'hydrogène ou méthyle, ou R¹⁰ et R¹¹ représentent ensemble cyclopropyle.

3. Composés selon la revendication 1 ou 2
où
R³ à R⁷, R¹⁰ et R¹¹ présentent la signification indiquée et
R¹, R², identiques ou différents, représentent l'hydrogène ou un groupement choisi dans le groupe consistant en C₁-C₆-alkyle, C₂-C₆-alcényle et C₂-C₆-alcynyle éventuellement substitué,
ou
R¹ et R² représentent ensemble un pont alkyle à 2 à 5 chaînons.

4. Composés selon l'une des revendications 1 à 3
où
R¹, R³ à R⁷, R¹⁰ et R¹¹ présentent la signification indiquée,
et
R³ représente l'hydrogène ou un groupement choisi dans le groupe consistant en C₁-C₁₂-alkyle, C₂-C₁₂-alcényle, C₂-C₁₂-alcynyle et C₆-C₁₄-aryle éventuellement substitué, ou
un groupement choisi dans le groupe consistant en C₃-C₁₂-cycloalkyle, C₃-C₁₂-cycloalcényle, C₇-C₁₂-polycycloalkyle, C₇-C₁₂-polycycloalcényle et C₅-C₁₂-spirocycloalkyle éventuellement substitué et/ou ponté.

5. Composés selon l'une des revendications 1 à 4
où
R¹ à R³, R⁵ à R⁷, R¹⁰ et R¹¹ présentent la signification indiquée
et
R⁴ représente un groupement de formule générale 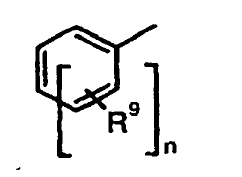 où
R⁹, identiques ou différents, représentent un groupement choisi dans le groupe consistant en C₁-C₆-alkyle, C₂-C₆-alcényle, C₂-C₆-alcynyle, -O-C₁-C₆-alkyle, -OC₂-C₆-alcényle, -O-C₂-C₆-alcynyle, C₃-C₆-hétérocycloalkyle, C₃-C₆-cycloalkyle, aryle, hétéroaryle, -O-aryle, -O-hétéroaryle, -O-cycloalkyle et -O-hétérocycloalkyle éventuellement substitué ou
un groupement choisi dans le groupe consistant en l'hydrogène, -CONH₂, -COOR⁸, -OCON(R⁸)₂, -N(R⁸)₂, -NHCOR⁸, -NHCON(R⁸)₂, -NO₂, CF₃, halogène, -O-C₁-C₆-alkyl-Q¹, -CONR⁸-C₁-C₁₀-alkyl-Q¹, -CONR⁸-C₂-C₁₀-alcényl-Q¹, -CONR⁸-Q², -OH, -SO₂R⁸, -SO₂N(R⁸)₂, -COR⁸, -COOR⁸, -CONR⁸OC₁-C₁₀-alkyl-Q¹ et CONR⁸O-Q²,
R⁸ représente dans chaque cas indépendamment l'hydrogène ou un groupement choisi dans le groupe consistant en C₁-C₄-alkyle, C₂-C₄-alcényle, C₂-C₄-alcynyle et phényle éventuellement substitué,
Q¹ représente l'hydrogène, -NHCOR⁸ ou un groupement choisi dans le groupe consistant en un groupe -NH-aryle, -NH-hétéroaryle, aryle, hétéroaryle, C₃-C₈-cycloalkyle et hétérocycloalkyle éventuellement substitué,
Q² représente l'hydrogène ou un groupement choisi dans le groupe consistant en un groupe aryle, hétéroaryle et C₃-C₈-cycloalkyle éventuellement substitué,
et
n représente 0, 1, 2, 3, 4 ou 5.

6. Composés selon l'une des revendications 1 à 5
où
Q¹, Q², n, R⁴ à R⁸ présentent la signification indiquée,
R¹, R², identiques ou différents, représentent l'hydrogène ou un groupement choisi dans le groupe consistant en méthyle, éthyle, propyle, allyle et propargyle
ou
R¹ et R² représentent ensemble cyclopropyle,
R³ est l'hydrogène ou C₁-C₆-alkyle éventuellement substitué ou C₃-C₁₂-cycloalkyle éventuellement substitué et/ou ponté, et
R¹⁰ et R¹¹ représentent l'hydrogène.

7. Composés selon la revendication 5 ou 6
où
Q¹, Q², n, R¹ à R⁴, R⁶ à R⁸, R¹⁰ et R¹¹ présentent la signification indiquée,
et
R⁹, identiques ou différents, représentent l'hydrogène ou un groupement choisi dans le groupe consistant en halogène, (C₁-C₄-alkyle)₂N-, CF₃, NH₂SO₂-, -CONH-C₆-C₁₄-aryle, -CONH-C₁-C₄-alkyl-C₆-C₁₄-aryle et -O-C₁-C₄-alkyle.

8. Composé de formule générale (I) selon l'une des revendications 1 à 7 destiné à être utilisé comme médicament.

9. Composé de formule générale (I) selon l'une des revendications 1 à 7 destiné à être utilisé comme médicament à action antiproliférative.

10. Utilisation d'un composé de formule générale (I) selon l'une des revendications 1 à 7 pour la production d'un médicament pour le traitement et/ou la prévention de maladies choisies dans le groupe consistant en le cancer, les infections bactériennes et virales, les maladies inflammatoires et auto-immunes, l'alopécie et l'inflammation des muqueuses induites par des agents chimiothérapeutiques, les maladies cardiovasculaires, les maladies néphrologiques ainsi que les maladies neurodégénératives chronique et aiguës.

11. Utilisation d'un composé de formule générale (I) selon l'une des revendications 1 à 7 pour la production d'un médicament pour l'inhibition des polo-like kinases.

12. Utilisation selon la revendication 11 **caractérisée en ce que** la polo-like kinase est PLK1.

13. Utilisation d'un composé de formule générale (I) selon l'une des revendications 1 à 7 pour la production d'un médicament pour le traitement et/ou la prévention des maladies tumorales dues à une surexpression des polo-like kinases.

14. Préparations pharmaceutiques contenant comme principe actif un ou plusieurs composés de formule générale (I) selon l'une des revendications 1 à 7 éventuellement en combinaison avec des adjuvants et/ou supports habituels.
